# FASCICULE DE BREVET EUROPEEN

(11) **EP 2 978 396 B1**
(45) Date de publication et mention de la délivrance du brevet: **10.10.2018**
(21) Numéro de dépôt: 14718670.4
(22) Date de dépôt: 25.03.2014
(51) Int. Cl.: A61F 13/42

(54) **DISPOSITIF ELECTRONIQUE AUTONOME DE DETECTION ET D'AVERTISSEMENT DE LA SATURATION D'UN ARTICLE DE TYPE COUCHE DE PROTECTION DESTINEE A L'INCONTINENCE UROFECALE**
AUTONOME ELEKTRONISCHE VORRICHTUNG ZUR ERKENNUNG UND MELDUNG DER SÄTTIGUNG EINES SCHUTZSCHICHTARTIKELS FÜR HARN- UND STUHLINKONTINENZ
SELF-CONTAINED ELECTRONIC DEVICE FOR DETECTING AND ALERTING OF THE SATURATION OF A PROTECTION LAYER ARTICLE FOR UROFECAL INCONTINENCE

(30) Priorité: 27.03.2013 FR 1300711
(43) Date de publication de la demande: 03.02.2016
(73) Titulaire: Geloen, Jacques, 59910 Bondues (FR)
(72) Inventeur: Geloen, Jacques, 59910 Bondues (FR)
(74) Mandataire: Oltmann, Eckhard
(86) Numéro de dépôt international: PCT/FR2014/050698
(87) Numéro de publication internationale: WO 2014/154998

(56) Documents cités:
- EP-A1- 0 270 048
- WO-A1-2007/070267
- WO-A1-2011/054045
- WO-A1-2011/126497
- WO-A1-2013/013197
- US-A1- 2010 122 391

## Description

La présente invention permet la visualisation à distance de la saturation d'une protection utilisée pour l'incontinence urofécale adulte et pédiatrique.

L'invention consiste en l'intégration d'un module générateur dans l'épaisseur absorbante d'une protection associé à un module témoin externe. Le dernier élément constituant le générateur de courant est situé à la frontière dorsale de la zone absorbante pour assurer une fonction d'interrupteur au dispositif lorsque la saturation est totale. Ce module génère un courant sous l'influence des urines acides au contact d'électrodes cuivre-zinc couplées. Ce courant, progressivement croissant mais délivré en tout ou rien, est capable d'assurer l'allumage soit d'une diode électroluminescente au chevet du patient soit d'activer une quelconque interface électronique ou informatique de gestion à distance.

Le but est d'objectiver avec certitude la nécessité du change pour éviter des problèmes cutanés chez des patients fragiles jeunes ou âgés. La problématique imposant le respect du sommeil selon l' « humanitude » en institution sera assurer, le soignant étant capable de voir le témoin au chevet du patient sans allumer la lumière de sa chambre et sans le découvrir pour observer les traits colorimétriques situés à l'entre cuisse et dans le dos. Un témoin allumé conduira au change d'une protection saturée. Un témoin éteint, lors de tour de nuit, imposera aux soignants de respecter le sommeil du patient.

La fréquence de l'incontinence en institution est estimée à 70 % des résidents. L'augmentation exponentielle de nos seniors laisse entrevoir l'incidence industrielle potentielle. L'ajout de ces modules générateur/ témoin aux protections améliorera nettement le confort de nos aînés comme des moins âgés.

A ce jour, les témoins de saturation utilisent une méthode chimique colorimétrique sous la forme de traits situés de l'entrecuisse à la région lombaire des patients. Les témoins virent du bleu au jaune au contact des urines. A l'évidence le contrôle visuel de ces témoins nécessite la mobilisation des patients avec le retrait des couvertures et un éclairage suffisant la nuit ce qui induit, par force, le réveil. On imagine les conséquences de ces vérifications nocturnes chez des résidents déments ou souffrant de troubles psychiatriques qui, une fois réveillés, n'ont aucune envie de se recoucher.

Par ailleurs, le principe d' « humanitude » impose le respect du sommeil chez les résidents vivant en institutions ce qui rend impossible la vérification des témoins colorimétriques durant les phases de sommeil nocturne comme diurne.

De plus cette problématique induit pour les soignants un dilemme qui partage les équipes avec :
- ceux qui respectent totalement les principes d' « humanitude » et qui ne réveillent pas les patients et donc ne vérifient pas les témoins de saturation au prix de complications cutanées graves (rougeurs, surinfections, mycoses, escarres) consécutives à la macération chez des patients noyés dans leurs urines.
- ceux qui vérifient systématiquement les témoins pour éviter les problèmes cutanés et réveillent les résidents, parfois inutilement, pour constater une protection sèche. Dans ce cas l'« humanitude » ne peut être respectée.

Il semble important également de signaler les rivalités induites par ces divergences de pratiques les uns accusant les autres de ne pas changer les patients pour reporter le travail sur l'équipe suivante, les autres s'offensant de réveiller les patients qui ensuite ne dorment plus.

Un grand nombre de publications montrent déjà des indicateurs d'humidité électroniques dans le contexte des produits d'hygiène absorbants.

WO2011126497A1 décrit un dispositif de surveillance pour un tampon avec un capteur qui est de préférence un capteur à résistance (capteur résistif).

Les indicateurs d'humidité électroniques décrits dans EP0270048A1 et WO2007070267A1 comprennent une paire d'électrodes non décrits plus en détail. Un circuit électronique détecte la connexion électrique entre les électrodes en cas de saturation de liquide.

WO2011054045A1 montre un système d'information assisté par ordinateur, qui comprend entre autres un capteur électronique qui fournit des informations sur l'entrée de liquide dans une couche-culotte. Le capteur est de préférence un capteur résistif.

D'autres documents précédents sont WO2011126497, EP0270048A1, WO2007070267A1 WO2011054045A1.

US20100122391A1 décrit un indicateur d'humidité électronique, dans lequel l'indicateur comprend une paire d'électrodes ayant un potentiel d'oxydoréduction, de sorte qu'un courant peut être généré en cas de l'entrée de liquide.

WO2013013197A1 montre également un indicateur d'humidité électronique, dans lequel l'indicateur comprend une paire d'électrodes ayant un potentiel d'oxydoréduction de sorte qu'un courant peut être généré en cas de saturation de liquide.

Le but de l'invention sera de visualiser la saturation de la protection, sans réveiller le patient, par le biais d'un témoin visuel, sonore, mécanique ou hertzien déporté à distance du patient. Dès lors, le change sera réalisé à bon escient lorsque le témoin sera activé, indiquant une protection totalement saturée, chez les patients ciblés.

Le générateur de courant est composé d'un arrangement de couples métalliques zinc-cuivre reliés en série et séparés les uns des autres par un espace assurant l'autonomie des couples en évitant toute interférence électrique entre eux. La disposition originale du générateur de courant inclus dans l'épaisseur de la couche absorbante de la protection a été choisi afin de créer une tension progressivement croissante et délivrée, pour activer le témoin, en on-off lorsque la saturation est totale et uniquement dans ce cas. Le soignant réveillera le résident pour faire un change nécessaire évitant ainsi tout problème cutané potentiel. Le principe d' « humanitude » sera respecté car seuls les patients ayant une protection saturée seront changés, les autres dont le témoin reste inactif poursuivront leur nuit normalement leur protection n'étant alors pas totalement saturée.

Le principe de cet indicateur externe évitera au soignant le contrôle des témoins situés dans le dos des patients le générateur de courant assurant cette fonction étant placé, pour partie, en regard de cette zone. En choisissant un indicateur visuel lumineux le respect du sommeil sera préservé en particulier la nuit lors du passage des aides-soignantes. Dans ce cadre, les règles d'« humanitude » sont respectées.

Par ailleurs, on imagine déjà les économies potentielles réalisables dès lors que le change sera réalisé à totale saturation, ce qui n'est pas le cas actuellement, car le réveil et le contrôle en pleine nuit débouche souvent sur le change d'une protection au quart ou à moitié saturée.

L'invention concerne un dispositif électronique autonome de détection et d'avertissement de la saturation d'un article de type couche de protection destinée à l'incontinence urofécale comprenant au moins un module générateur électrique autonome comportant un arrangement de couples d'éléments à électrode métallique reliés électriquement entre eux et répartis sur une zone prédéterminée interne de l'article, de type notamment zinc - cuivre, destiné à générer un courant au contact d'un milieu urofécal acide et aqueux par réaction d'oxydoréduction, pour commander électriquement de manière autonome des moyens internes ou externes d'avertissement de la saturation notamment de type lumineux, sonore, de transmission analogique ou numérique de signaux avec fil ou sans fil...

Selon des modes de réalisation particuliers :
- les couples d'éléments à électrode métallique conformés sont reliés en série électriquement avec une distance entre chaque couple minimale évitant les interactions, les bornes de connexion de sortie étant la première électrode du premier couple et la seconde électrode du dernier couple.
- chaque élément d'électrode en bandes parallèles d'un couple étant séparé l'un de l'autre par un espace parallèle recevant le milieu urofécal, assurant l'autonomie des couples en évitant toute interférence électrique entre eux.
- le dernier couple d'électrodes de l'arrangement est situé à la partie frontière et dorsale de la protection pour assurer une fonction « interrupteur tout ou rien » à l'ensemble du générateur lorsque ce dernier élément est mouillé pour indiquer la saturation de l'article de type couche de protection.
- l'ensemble est intégré en tout ou partie dans une enveloppe textile comprenant des pores dans des zones notamment en regard des espaces séparant les électrodes.
- le module générateur électrique comprend une structure souple de faible épaisseur de type carte de circuit imprimé à une ou plusieurs faces de pistes conductrices de un ou plusieurs matériaux.
- les moyens d'avertissement comprennent au moins une diode électroluminescente interne ou externe alimentée uniquement par le module générateur.
- les moyens d'avertissement comprennent au moins une source lumineuse interne de type diode électroluminescente associée optiquement à un moyen type fibres optiques pour la diffusion éloignée de l'information.
- le module générateur électrique s'étend dans l'axe central longitudinal de l'article de type couche de protection destinée à l'incontinence avec au moins une majeure partie située vers la partie arrière de l'article.
- le module générateur électrique a une conformation en bande (notamment de 30 cm) constituée d'un arrangement de couples d'électrodes perpendiculaires et symétriques par rapport à l'axe centrale (notamment de 2 cm de part et d'autre), les couples étant espacés entre eux d'une distance prédéterminée (notamment de 4 cm)

L'invention se compose de deux parties :
- un générateur de courant intégré dans l'épaisseur absorbante de la protection ayant la particularité technique d'induire une tension croissante en marches d'escalier au fur et à mesure de la progression de l'imbibition .Cependant cette tension générée ne sera exploitée aux bornes du système uniquement lors de l'imprégnation totale de la protection témoignant la saturation complète.
- un témoin externe, visuel dans cette version, placé à distance du patient et relié au module générateur par un fin fil conducteur bipolaire isolé. Des variantes sont envisagées plus loin dans la description.

Le système consiste à générer un courant créé par un arrangement de couples métalliques, ou unités, en série régulièrement espacés formant des piles zinc-cuivre plongées dans un milieu acide et aqueux. Les électrodes du générateur, en milieu acide, seront le siège d'une réaction d'oxydoréduction des métaux ceux-ci étant de natures différentes .On peut imaginer une multitude de couples métalliques pouvant être à l'origine d'une telle réaction d'oxydoréduction en milieu acide. Cependant, de façon empirique, le couple zinc-cuivre donne les meilleurs résultats en terme de force électromotrice. Ce générateur, disposé et inclus dans la couche absorbante de la protection, allumera une diode électroluminescente DEL ou un autre témoin. Les urines constituent la solution idéale acide avec un PH oscillant entre 4 et 6. Les unités, constituées chacune d'un fil plat de cuivre et d'un autre en zinc parallèles et séparés par un fin vide sont fixées sur un support textile ou autre assurant le maintien des électrodes ainsi que l'espace résistif nécessaire entre couple. Une liaison isolée est ensuite réalisée, entre les unités, par un conducteur gainé pour aboutir à un générateur produisant un courant avoisinant 2 volts et quelques milliampères nécessaire à la LED.

La dernière unité, placée à la partie dorsale et frontière de la couche absorbante, ultime unité imprégnée par les urines, remplira le rôle de contacteur induisant l'activation du témoin. De par cette disposition, le témoin ne pourra s'activer que si, et seulement si, le dernier élément est mouillé, donc conducteur, ce qui évitera des changes de protections à moitié saturées. Le courant produit est progressivement croissant par l'imprégnation de proche en proche des éléments du générateur puis enfin délivré lorsque le dernier élément est mouillé constituant le on-off autonome du système.

Pour des raisons économiques un témoin lumineux sera retenu pour cet exposé mais toutes autres interfaces peut être employées : sonores, informatiques, mécaniques, hertziennes...

Dans l'exemple, Le témoin filaire constitué par la diode électroluminescente, son fil conducteur bipolaire et le connecteur miniature le reliant au générateur sera installé au chevet du patient. La diode LED sera placée à distance du patient pour être visible par le soignant qui réalisera son tour de nuit. Le connecteur miniature utilisé est placé sur la face avant de la protection afin d'éviter toute blessure cutanée par pression lors du sommeil.

Le dispositif témoin pourra être réutilisé dans sa version stérilisable pour réduire au maximum les coûts. Les matériaux : fils, jonctions, contacteurs sont souples et inoffensifs pour éviter toute blessure ou mauvaise manipulation.

La diode électroluminescente (DEL) déportée du générateur peut également être incorporée dans la protection en regard du connecteur. Dans ce cas, le transfert des photons émis par la diode sera assuré par une fibre optique fixée sur la diode dont l'extrémité sera placée à distance du patient. La source lumineuse se trouve alors intégrée à la protection et véhiculée à distance par la fibre optique au chevet du patient. Cette méthode, très économique, simplifiera les manipulations de connexion.

Selon les modes de fabrication des variantes peuvent être envisagées :
- d'autres métaux peuvent être utilisés pour élaborer les éléments du générateur de courant. Cependant le couple cuivre-zinc semble le plus approprié avec un rendement et une force électromotrice supérieurs aux couples aluminium- cuivre ou plomb-cuivre en milieu acide. Des unités chimiques peuvent également être envisagées pour produire l'énergie nécessaire.
L'arrangement induira une tension croissante, type marches d'escalier, en fonction de l'imbibition progressive des couples.
- le nombre de cellules peut varier en fonction de la tension et de l'intensité souhaitée. De même la surface des électrodes métalliques en contact avec le milieu acide et la distance inter électrodes conditionnent le courant final fourni. Concernant le générateur, l'utilisation d'une fine lame souple époxy (type circuit imprimé) intégrant l'électrode cuivre (piste de cuivre) et l'électrode zinc (collée sur le support époxy) séparé par un espace le plus petit possible semble apporter une solution facilitant une production industrielle lors de l'assemblage des éléments du générateur. Les couples zinc-cuivre seront reliés entre eux par une piste isolée par un verni. L'inhibition des électrodes du couple sera assurée par contact directe avec le matériau absorbant de la protection plaqué sur la lame époxy. Ce générateur souple, donc compatible aux mouvements du corps, induira une tension croissante, type marches d'escalier, en fonction de l'imbibition progressive des couples. La fonction on-off du dernier élément situé en regard de la région dorsale est conservé assurant l'activation du témoin externe lumineux, hertzien, informatique, mécanique ou autre.
- la diode électroluminescente polarisée peut être remplacée par tout autre dispositif électronique tel un circuit déclencheur, type bascule JK CMOS ou un ampli opérationnel, capable de commander une quelconque interface informatique, hertzienne ou mécanique (relais). Le courant fourni peut également servir à la commande d'un module émetteur hyperfréquence téléphonique intégré dans la protection. Le coût final sera bien entendu le paramètre principal limitant les possibles extensions.
- l'urine et /ou l'eau des selles sont les éléments fondamentaux nécessaires à l'activation du générateur constitué d'un arrangement de couples cuivre-zinc. Le PH doit être acide. Dans l'hypothèse d'un PH urinaire neutre ou alcalin, l'adjonction sous forme pulvérulente d'un acide faible oxalique, tartrique ou citrique au contact des électrodes des couples suppléera ce défaut lors de sa dissolution aqueuse. La réalisation se fera par simple trempage des électrodes dans l'acide faible pulvérulent durant la phase de montage sur le support textile ou époxy souple.
- les électrodes métalliques constituant le générateur seront maintenues soit par l'utilisation d'un film textile souple semi-perméable, éventuellement adhésif, soit sur un circuit imprimé époxy souple. Des pores seront réalisés en regard des espaces séparant les électrodes métalliques dans la version textile inutiles dans la version circuit imprimé. Ces orifices perforés, lors de la fabrication, laisseront passer les urines pour imbiber au mieux les couples cuivre-zinc.
- le textile semi-perméable évite les interactions entre les éléments constitutifs du générateur. En effet, des éléments placés trop près, baignant dans une solution conductrice, aboutirait à la neutralisation de l'effet pile les uns par rapport aux autres. Il est donc nécessaire d'espacer les éléments contenant les couples cuivre-zinc par une distance suffisante donc résistive. De plus, cet espacement est utile pour obtenir une progressivité en fonction de l'imprégnation de la protection. Signalons à nouveau le rôle fondamental de la dernière unité comme conducteur déclencheur à l'allumage du témoin lumineux lors de la saturation totale dorsale du change.
L'utilisation d'un circuit imprimé souple, à la place du textile, gravé des électrodes cuivrées et des pistes de jonction isolées couplées aux électrodes zinc collées, formant les unités, apportera d'emblée l'espacement résistif entre couples sans que les électrodes puissent bouger car gravées et maintenues sur le circuit imprimé.

Les dessins annexés illustrent l'invention :

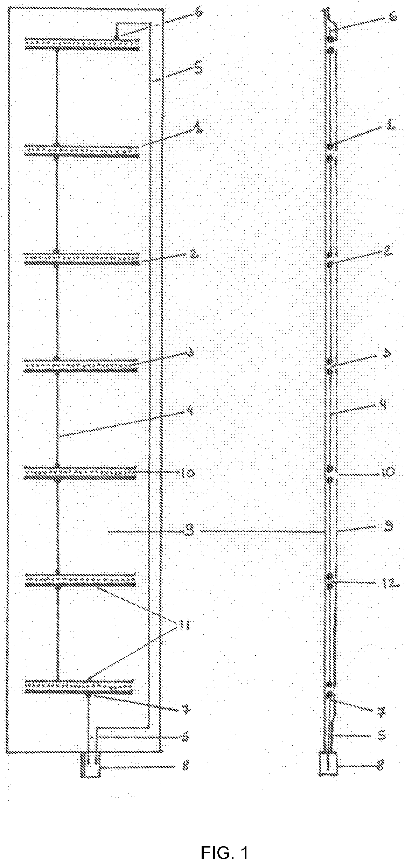

- la FIGURE 1 représente en coupe et en vue de dessus le module générateur avec ses éléments. Les éléments sont composés d'un fil plat de cuivre (1) séparé du fil plat de zinc (2) par un espace très fin (3). Un fil conducteur gainé est soudé pour assurer la jonction électrique des éléments entre eux (4). Aux extrémités du générateur des fils conducteurs (5) gainés sont soudés aux électrodes finales, cuivre d'un côté (6) zinc de l'autre (7) pour être amenés au connecteur externe de la protection (8). Les éléments sont fixés sur le textile souple semi-perméable (9). Des orifices perforés permettent l'imbibition de l'espace inter électrodes (10). La distance (11) séparant les éléments couplés permet d'éviter une interaction entre ceux-ci annulant l'effet pile. L'adjonction d'un acide faible pulvérulent (12) dans l'espace inter électrodes est possible pour assurer un PH bas en dissolution par les urines trop neutres ou faiblement basiques.

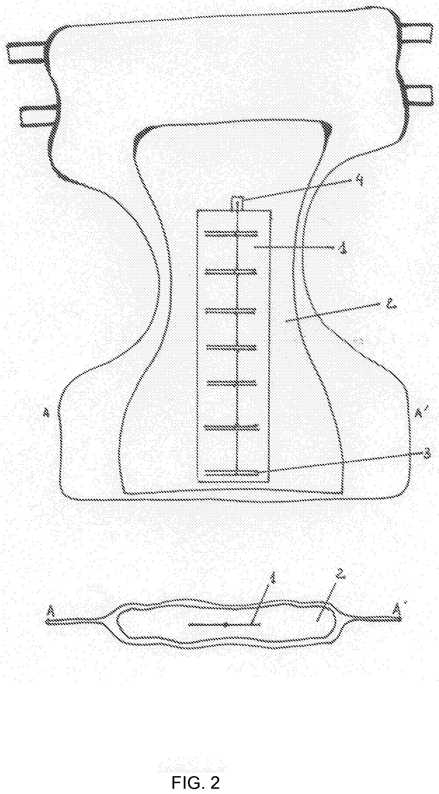

- la FIGURE 2 représente une protection en coupe et de dessus contenant le module générateur de courant. Le générateur (1) est intégré dans la couche absorbante de la protection (2). Le dernier élément du générateur (3) est disposé à la frontière dorsale de la partie absorbante. Un connecteur électrique (4) polarisé dépasse de la protection sur la face avant de celle-ci en regard de la région hypogastrique anatomique.

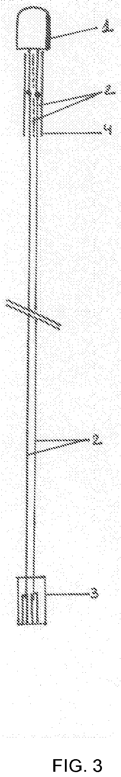

- la FIGURE 3 représente le témoin filaire. La diode électroluminescente (1) est soudée à l'une des extrémités du fil conducteur bipolaire (2). Un connecteur (3) est soudé à l'autre extrémité du fil conducteur. L'isolation des soudures est réalisée par du retrain thermo déformable (4).

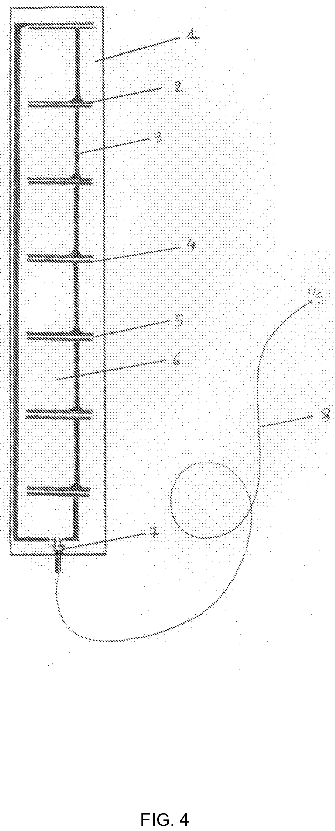

- la FIGURE 4 illustre la version sur circuit imprimé. Un circuit imprimé époxy de très faible épaisseur (1) est gravé de pistes électrodes (2) non isolées. Cette même face possède les pistes de jonction isolées par un verni (3) reliant les électrodes en série, jouxtant les électrodes cuivrées sont collées les électrodes zinc (4) réalisées par un fin fil ou par une peinture conductrice à base de zinc (galvanisation). Un espace minime est laissé entre les électrodes (5) afin que les urines acides baignent les métaux pour induire l'oxydoréduction attendue. De même, la distance inter unité électrochimique (6), modulable à souhait pour obtenir la résistance nécessaire à l'isolation des couples, s'avère facilité avec l'époxy qui est un excellent isolant. A l'extrémité dans cette version un témoin électroluminescent (7) type LED sera soudé sur le circuit imprimé avec la possibilité de brancher une fibre optique (8) qui cheminera les photons à distance du patient. Tout autre dispositif témoin peut être envisagé comme cité précédemment.

A titre d'exemple, non limitatif, les dimensions du module générateur sur textile souple seront : 30 centimètres de longueur sur 5 centimètres de largeur et 0.01 centimètre d'épaisseur. Les électrodes sont réalisées en fil plat de 0.50 millimètre de côté sur 4.5 centimètres de longueur. L'espace inter électrodes sera le plus petit possible sans contact. La distance entre les éléments de la pile sera de 4 centimètres minimum pour éviter les interférences et obtenir une gradation de la montée en tension selon la saturation.

## Revendications

1. Dispositif électronique autonome de détection et d'avertissement de la saturation d'un article de type couche de protection destinée à l'incontinence urofécale **caractérisé en ce qu'**il comprend au moins un module générateur électrique autonome comportant un arrangement de couples d'éléments à électrode métallique reliés électriquement entre eux et répartis sur une zone prédéterminée interne de l'article, chaque couple d'éléments à électrode, notamment de type zinc - cuivre, étant apte à générer un courant au contact d'un milieu urofécal acide et aqueux par réaction d'oxydoréduction, pour commander électriquement de manière autonome des moyens internes ou externes d'avertissement de la saturation de type lumineux et/ou sonore.

2. Dispositif selon la revendication 1 **caractérisé en ce que** les couples d'éléments à électrode métallique conformés sont reliés en série électriquement avec une distance entre chaque couple minimale évitant les interactions, les bornes de connexion de sortie étant la première électrode du premier couple et la seconde électrode du dernier couple.

3. Dispositif selon la revendication 1 ou 2 **caractérisé en ce que** chaque élément d'électrode en bandes parallèles d'un couple étant séparé l'un de l'autre par un espace parallèle recevant le milieu urofécal, assurant l'autonomie des couples en évitant toute interférence électrique entre eux.

4. Dispositif selon l'une quelconque des revendications précédentes **caractérisé en ce que** le dernier couple d'électrodes de l'arrangement est situé à la partie frontière et dorsale de la protection pour assurer une fonction « interrupteur tout ou rien » à l'ensemble du générateur lorsque ce dernier élément est mouillé pour indiquer la saturation de l'article de type couche de protection.

5. Dispositif selon l'une quelconque des revendications précédentes **caractérisé en ce que** l'ensemble est intégré en tout ou partie dans une enveloppe textile comprenant des pores dans des zones notamment en regard des espaces séparant les électrodes.

6. Dispositif selon l'une quelconque des revendications précédentes **caractérisé en ce que** le module générateur électrique comprend une structure souple de faible épaisseur de type carte de circuit imprimé à une ou plusieurs faces de pistes conductrices de un ou plusieurs matériaux.

7. Dispositif selon l'une quelconque des revendications précédentes **caractérisé en ce que** les moyens d'avertissement comprennent au moins une diode électroluminescente interne ou externe alimentée uniquement par le module générateur.

8. Dispositif selon l'une quelconque des revendications précédentes **caractérisé en ce que** les moyens d'avertissement comprennent au moins une source lumineuse interne de type diode électroluminescente associée optiquement à un moyen à fibres optiques pour la diffusion éloignée de l'information.

9. Dispositif selon l'une quelconque des revendications précédentes **caractérisé en ce que** le module générateur électrique s'étend dans l'axe central longitudinal de l'article de type couche de protection destinée à l'incontinence avec au moins une majeure partie située vers la partie arrière de l'article.

10. Dispositif selon l'une quelconque des revendications précédentes **caractérisé en ce que** le module générateur électrique a une conformation en bande de préférence de 30 cm constituée d'un arrangement de couple d'électrodes perpendiculaires et symétriques par rapport à l'axe centrale de préférence de 2 cm de part et d'autre, les couples étant espacée entre eux d'une distance prédéterminée de préférence de 4 cm.

## Patentansprüche

1. Autonome elektronische Vorrichtung zur Erfassung und Meldung der Sättigung eines Artikels von der Art einer für die Harn- und Stuhlinkontinenz bestimmten Schutzwindel, **dadurch gekennzeichnet, dass** sie mindestens ein autonomes elektrisches Generatormodul enthält, das eine Anordnung von Paaren von Elementen mit Metallelektrode aufweist, die elektrisch miteinander verbunden und über einen vorbestimmten inneren Bereich des Artikels verteilt sind, wobei jedes Paar von Elementen mit Elektrode, insbesondere von der Art Zink-Kupfer, in Kontakt mit einem sauren und wässrigen Harn-Stuhl-Medium durch Redoxreaktion einen Strom erzeugen kann, um autonom innere oder äußere leuchtende und/oder akustische Einrichtungen zur Meldung der Sättigung elektrisch zu steuern.

2. Vorrichtung nach Anspruch 1, **dadurch gekennzeichnet, dass** die ausgebildeten Paare von Elementen mit Metallelektrode mit einem Wechselwirkungen vermeidenden minimalen Abstand zwischen jedem Paar elektrisch in Reihe geschaltet sind, wobei die Ausgangsanschlussklemmen die erste Elektrode des ersten Paars und die zweite Elektrode des letzten Paars sind.

3. Vorrichtung nach Anspruch 1 oder 2, **dadurch gekennzeichnet, dass** jedes Elektrodenelement in parallelen Streifen eines Paars voneinander durch einen parallelen Raum getrennt ist, der das Harn-Stuhl-Medium aufnimmt, was die Autonomie der Paare gewährleistet, indem jede elektrische Interferenz zwischen ihnen vermieden wird.

4. Vorrichtung nach einem der vorhergehenden Ansprüche, **dadurch gekennzeichnet, dass** das letzte Paar von Elektroden der Anordnung sich am Grenz- und Rückenteil des Schutzes befindet, um eine "Ein-AusSchalter"-Funktion der Gesamtheit des Generators zu gewährleisten, wenn letzteres Element feucht wird, um die Sättigung des Artikels von der Art Schutzwindel anzuzeigen.

5. Vorrichtung nach einem der vorhergehenden Ansprüche, **dadurch gekennzeichnet, dass** die Einheit ganz oder teilweise in eine Textilhülle integriert ist, die in Bereichen insbesondere gegenüber den die Elektroden trennenden Räumen Poren enthält.

6. Vorrichtung nach einem der vorhergehenden Ansprüche, **dadurch gekennzeichnet, dass** das elektrische Generatormodul eine biegsame Struktur geringer Dicke von der Art Leiterplatte mit einer oder mehreren Seiten von Leiterbahnen aus einem oder mehreren Materialien enthält.

7. Vorrichtung nach einem der vorhergehenden Ansprüche, **dadurch gekennzeichnet, dass** die Meldeeinrichtungen mindestens eine innere oder äußere Elektrolumineszenzdiode enthalten, die nur vom Generatormodul gespeist wird.

8. Vorrichtung nach einem der vorhergehenden Ansprüche, **dadurch gekennzeichnet, dass** die Meldeeinrichtungen mindestens eine innere Lichtquelle von der Art Elektrolumineszenzdiode enthalten, die optisch einer Einrichtung mit Lichtleitfasern zur Fernübermittlung der Information zugeordnet ist.

9. Vorrichtung nach einem der vorhergehenden Ansprüche, **dadurch gekennzeichnet, dass** das elektrische Generatormodul sich in der Längsmittelachse des Artikels von der Art einer für die Inkontinenz bestimmten Schutzwindel mit mindestens einem größeren Teil zum hinteren Teil des Artikels hin befindlich erstreckt.

10. Vorrichtung nach einem der vorhergehenden Ansprüche, **dadurch gekennzeichnet, dass** das elektrische Generatormodul eine Ausbildung in Form eines Streifens von vorzugsweise 30 cm hat, der aus einer paarweisen Anordnung von Elektroden lotrecht und symmetrisch bezüglich der Mittelachse vorzugsweise von 2 cm auf beiden Seiten besteht, wobei die Paare einen vorbestimmten Abstand zueinander von vorzugsweise 4 cm haben.

## Claims

1. Autonomous electronic device for detecting and signaling the saturation of a protective liner type article intended for urofecal incontinence, **characterized in that** it comprises at least one autonomous electrical generator module comprising an arrangement of pairs of metal electrode elements that are electrically connected to one another and distributed over a preset internal zone of the article, each pair of especially zinc/copper electrode elements being able to generate a current on contact with an aqueous and acid urofecal medium via a redox reaction, in order to electrically and autonomously control internal or external means for signaling the saturation, these means being luminous and/or sonic.

2. Device according to Claim 1, **characterized in that** the pairs of formatted metal electrode elements are connected in series electrically with a minimum distance between each pair preventing interactions, the output connection terminals being the first electrode of the first pair and the second electrode of the last pair.

3. Device according to Claim 1 or 2, **characterized in that** each electrode element made up of parallel strips of a pair being separated one from the other by a parallel space receiving the urofecal medium, ensuring the autonomy of the pairs by preventing any electrical interference between them.

4. Device according to any one of the preceding claims, **characterized in that** the last pair of electrodes of the arrangement is located in the dorsal boundary portion of the protection in order to provide the generator assembly with an "on/off switch" function that is activated when the latter element is wetted in order to indicate the saturation of the protective liner type article.

5. Device according to any one of the preceding claims, **characterized in that** the assembly is completely or partially integrated into a textile envelope comprising pores in zones especially facing the spaces separating the electrodes.

6. Device according to any one of the preceding claims, **characterized in that** the electrical generator module comprises a thin flexible printed circuit board type structure comprising one or more sides of conductive tracks of one or more materials.

7. Device according to any one of the preceding claims, **characterized in that** the signaling means comprise at least one internal or external light-emitting diode powered only by the generator module.

8. Device according to any one of the preceding claims, **characterized in that** the signaling means comprise at least one internal light source of the light-emitting diode type associated optically with an optical-fiber-comprising means for transmitting the information over distance.

9. Device according to any one of the preceding claims, **characterized in that** the electrical generator module extends along the longitudinal central axis of the protective liner type article intended for incontinence with at least one major portion located toward the back portion of the article.

10. Device according to any one of the preceding claims, **characterized in that** the electrical generator module has a strip format preferably of 30 cm consisting of an arrangement of electrode pairs perpendicular and symmetric relative to the central axis preferably of 2 cm on either side, the pairs being spaced apart by a preset preferably distance of 4 cm.
